# DEMANDE DE BREVET EUROPEEN

(11) **EP 2 543 392 A1**
(43) Date de publication de la demande: **09.01.2013**
(21) Numéro de dépôt: 12175255.4
(22) Date de dépôt: 06.07.2012
(51) Int. Cl.: A61L 2/26, B25G 1/00, B65D 25/28

(54) **Panier de sterilisation avec porte-panier**

(30) Priorité: 07.07.2011 FR 1156179
(71) Demandeur: Vygon, 95440 Ecouen (FR)
(72) Inventeur: Lestoquoy, Patrick, 59551 ATTICHES (FR); Guermache, Nordine, 95260 BEAUMONT SUR OISE (FR)
(74) Mandataire: Regimbeau

(57) **Abrégé**

L'invention concerne un panier de stérilisation (1), caractérisé en ce qu'il est équipé d'un porte-panier (20) comprenant des moyens d'accrochage (22) au panier de stérilisation (1) et une poignée de préhension (24), ledit porte-panier (20) étant non solidaire du panier de stérilisation (1).

## Description

L'invention concerne de manière générale les paniers de stérilisation et leur utilisation en condition d'asepsie.

L'asepsie désigne l'absence de germes microbiens susceptibles d'entrainer l'apparition d'une infection. On désigne par faute d'asepsie une manoeuvre qui risque de souiller un champ stérile, un instrument, ou encore les gants stériles d'un chirurgien.

Le milieu chirurgical notamment doit respecter un grand nombre de règles d'asepsie, quelle que soit l'importance ou la gravité de l'intervention chirurgicale. Ainsi, dans ce milieu, l'asepsie désigne l'ensemble des méthodes qui permettent de préserver et de prévenir la survenue d'une infection microbienne. Elle est généralement obtenue en désinfectant l'ensemble de la zone d'intervention chirurgicale, grâce à l'utilisation d'antiseptiques, et grâce à la stérilisation préalable des instruments, gants, pansements, vêtements, etc. destinés à être utilisés lors de l'intervention. Par ailleurs, la zone opératoire est protégée par des champs opératoires stériles à usage unique.

Les instruments chirurgicaux sont généralement disposés dans des paniers de stérilisation, de préférence de manière ordonnée et organisée de manière déterminée par la pratique usuelle.

Afin de préserver la stérilité des instruments chirurgicaux, le panier est enveloppé selon le pliage « Pasteur » ou « américain » dans un emballage, typiquement une, et plus généralement deux feuilles d'emballage : une feuille d'emballage primaire, constituant une barrière microbienne autour des instruments médicaux et du panier de stérilisation et permettant la présentation aseptique des instruments sur leur point d'utilisation, et une feuille d'emballage secondaire, qui entoure la feuille d'emballage primaire et est conçue pour éviter tout dommage au système de barrière stérile et à son contenu depuis l'assemblage jusqu'au point s'utilisation.

On stérilise alors l'ensemble formé par le panier comportant les instruments chirurgicaux et l'emballage.

Avantageusement, les paniers de stérilisation ainsi emballés peuvent être conservés de manière stérile pendant plusieurs jours voire plusieurs semaines. Néanmoins, le déballage et l'extraction du panier de stérilisation des feuilles d'emballage est source de fautes d'asepsie. En effet, après stérilisation, le stockage du panier enveloppé des feuilles d'emballage est réalisé dans des endroits non-stériles, de sorte que la feuille secondaire (voire même la feuille primaire) entre en contact avec des germes microbiens et n'est donc plus aseptique. Par conséquent, lors de l'extraction du panier de stérilisation, l'opérateur doit absolument éviter que ses gants entrent en contact avec l'une ou l'autre des feuilles d'emballage.

Or, les feuilles d'emballage sont réalisées dans un matériau présentant une importante mémoire de forme après stérilisation, de sorte qu'au moment de l'extraction du panier, l'opérateur risque d'entrer en contact avec elles malgré sa vigilance. Si l'opérateur se rend compte de cette faute d'asepsie, il est alors nécessaire à la fois de changer les gants de l'opérateur et d'utiliser un autre panier de stérilisation, ce qui, en dehors d'engendrer des coûts importants, fait perdre du temps à l'opérateur.

On a donc proposé dans le document DE 10 2006 062 379 de munir le panier de stérilisation d'un porte-panier, comprenant deux anses fixées solidairement aux parois latérales du panier. Toutefois, la manipulation du panier reste sensible car il est difficile de saisir les poignées, qui sont souvent dissimulées par les feuilles d'emballage. L'opérateur risque donc de commettre une faute d'aseptie en cherchant à attraper les poignées.

Le document US 2010/0001013 quant à lui décrit une poignée amovible comprenant des moyens élastiques munis de crochets adaptée pour venir en prise avec un panier. Néanmoins, cette poignée n'est pas adaptée aux paniers de stérilisation, dans la mesure où il est nécessaire de la retirer pour accéder au contenu du panier. Or pour la retirer, il est nécessaire de tenir le panier afin de désengager les crochets en raison de la présence des moyens élastiques. Par ailleurs, cette poignée n'est nullement adaptée aux étapes de stérilisation qui doivent être subies par les paniers de stérilisation.

Enfin, le document US 2 812 968 décrit un porte-contenant amovible adapté pour être enfilé sur un contenant, tel qu'une bouteille. A nouveau, le contenant n'étant pas un panier de stérilisation, la poignée n'est pas adaptée pour subir des traitements de stérilisation. En outre, sa forme et sa structure sont difficilement adaptables à la saisie d'un panier de stérilisation. Enfin, les boucles du porte-contenant sont réalisées dans un matériau élastique, rendant ainsi très difficile pour un opérateur de retirer le porte-contenant du contenant sans commettre de faute d'aseptie.

L'invention a donc pour objet de pallier ces problèmes de l'état de la technique et de proposer un panier de stérilisation permettant de limiter les risques de faute d'asepsie, en particulier au moment de l'extraction du panier de stérilisation de son emballage, ainsi qu'un procédé d'utilisation d'un tel panier.

Pour cela, l'invention propose un panier de stérilisation, notamment pour un bloc opératoire, équipé d'un porte-panier comprenant des moyens d'accrochage au panier de stérilisation et une poignée de préhension, ledit porte-panier étant non solidaire du panier de stérilisation.

Certains aspects préférés mais non limitatifs du panier de stérilisation selon l'invention sont les suivants :
- les dimensions et le matériau constitutif du porte-panier sont choisis de sorte qu'il présente une résistance mécanique suffisante pour supporter le poids du panier de stérilisation et d'instruments médicaux ;
- il est réalisé dans un tissu tissé ou non-tissé ;
- les moyens d'accrochage comprennent une boucle fermée adaptée pour être enfilée autour du panier de stérilisation ;
- les moyens d'accrochage comprennent deux boucles fermées ;
- le porte-panier comprend en outre des bandes de maintien latérales s'étendant transversalement aux boucles fermées, adaptées pour maintenir le panier de stérilisation en position dans le porte-panier ; et
- la poignée de préhension comprend une bande fixée sur les moyens d'accrochage de manière à les relier ensemble et à former une anse.

Selon un deuxième aspect, l'invention propose également un ensemble formé d'un panier de stérilisation équipé d'un porte-panier non solidaire conforme à l'invention, et enveloppé dans un emballage.

Un aspect préféré mais non limitatif de cet ensemble conforme à l'invention est que le panier de stérilisation et le porte-panier sont complètement enveloppés dans l'emballage.

Selon un troisième aspect, l'invention propose un ensemble formé d'un panier de stérilisation équipé d'un porte-panier non-solidaire du panier conformes à l'invention, et comprenant en outre des instruments médicaux stériles logés dans le panier de stérilisation.

Selon un dernier aspect, l'invention propose un procédé d'utilisation d'un panier de stérilisation équipé d'un porte-panier non-solidaire du panier conforme à l'invention, ledit panier étant enveloppé dans un emballage, et comprenant les étapes suivantes :
- ouvrir l'emballage,
- saisir la poignée de préhension,
- tirer sur la poignée de préhension de manière à soulever le panier de stérilisation et à l'extraire de l'emballage, et
- poser le panier de stérilisation à un endroit voulu, en dehors de l'emballage.

Certains aspects préférés mais non limitatifs du procédé d'utilisation selon l'invention sont les suivants :
- l'emballage comprend une feuille d'emballage primaire et une feuille d'emballage secondaire ;
- le procédé comprenant en outre une étape au cours de laquelle le porte-panier est jeté après avoir posé le panier de stérilisation à l'endroit voulu ; et
- le procédé comprend en outre une étape au cours de laquelle le porte-panier est retiré du panier de stérilisation que celui-ci a été posé à l'endroit voulu, puis réutilisé pour porter un nouveau panier.

D'autres caractéristiques, buts et avantages de la présente invention apparaitront mieux à lecture de la description détaillée qui va suivre, donnée à titre d'exemple non-limitatif et faite en référence aux figures annexées données à titre non limitatif et sur lesquelles :
La figure 1 illustre une forme de réalisation d'un panier de stérilisation équipé d'un porte-panier conforme à l'invention, et
La figure 2 illustre une variante de réalisation de la figure 1.

Nous allons à présent décrire un exemple de panier de stérilisation 1 conforme à l'invention, en référence aux figures annexées.

Un tel panier de stérilisation 1 trouve application notamment dans le domaine chirurgical, où l'asepsie des instruments est primordiale. Ceci n'est cependant pas limitatif, dans la mesure où le panier 1 peut également être utilisé dans tout domaine médical nécessitant un stockage d'objets stériles.

Un panier de stérilisation 1 conforme à l'invention comprend un fond 14 depuis lequel s'étendent sensiblement perpendiculairement des parois latérales 12. La partie supérieure 16 du panier 1 en regard du fond 14 est de préférence ouverte, afin que son espace interne soit accessible depuis l'extérieur par un opérateur.

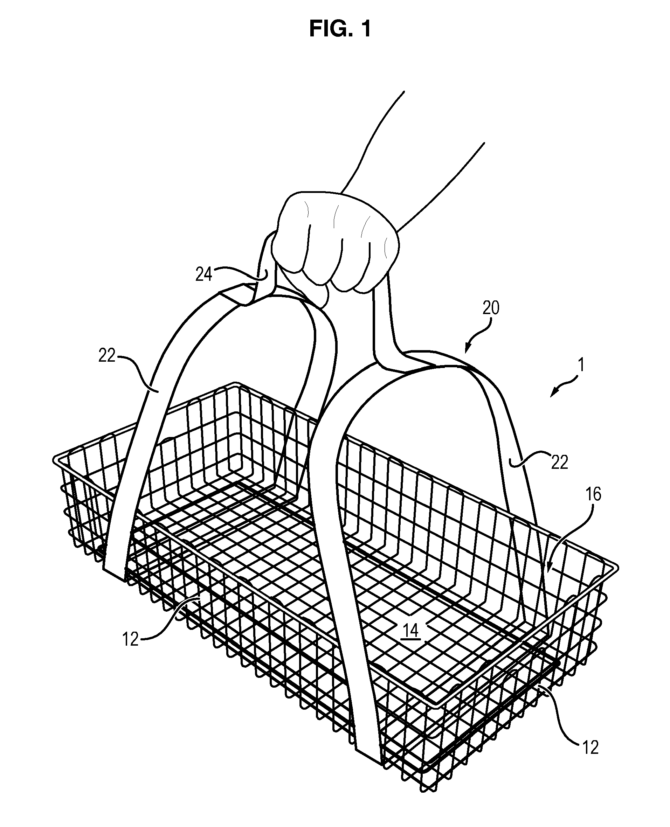

Le fond 14 et les parois latérales 12 peuvent être pleins ou ajourés. Par exemple, sur la figure 1, le fond 14 et les parois latérales 12 sont réalisés par maillage d'un fil rigide. Il peut être réalisé par exemple avec un fil métallique en acier inoxydable du type 18/10, selon une maille de 6 x 6 x 1.5 mm, et présenter des dimensions de l'ordre de 500 x 300 x 50 mm. ces dimensions ne sont bien entendu pas données à titre limitatif.

Par ailleurs, les formes du fond 14 et des parois latérales 12 sont de préférence adaptées pour que le panier 1 soit superposable une fois enveloppé dans l'emballage, c'est-à-dire apte à être empilé sur un autre panier 1 identique. Pour cela, les parois latérales 12 forment par exemple avec le fond 14 un angle proche mais supérieur à 90°, typiquement 100°.

Le panier de stérilisation 1 comporte en outre au moins un porte-panier 20, de préférence non-solidaire du panier 1, comprenant des moyens d'accrochage 22 au panier de stérilisation 1 et une poignée de préhension 24.

Sur la figure 1, les moyens d'accrochage 22 ont la forme de deux boucles fermées, adaptées pour être enfilées sur le panier de stérilisation 1 de manière à s'étendre transversalement à la partie supérieure 16 dudit panier 1, la poignée de préhension 24 s'étendant alors entre les deux moyens d'accrochage 22 de manière à former une anse.

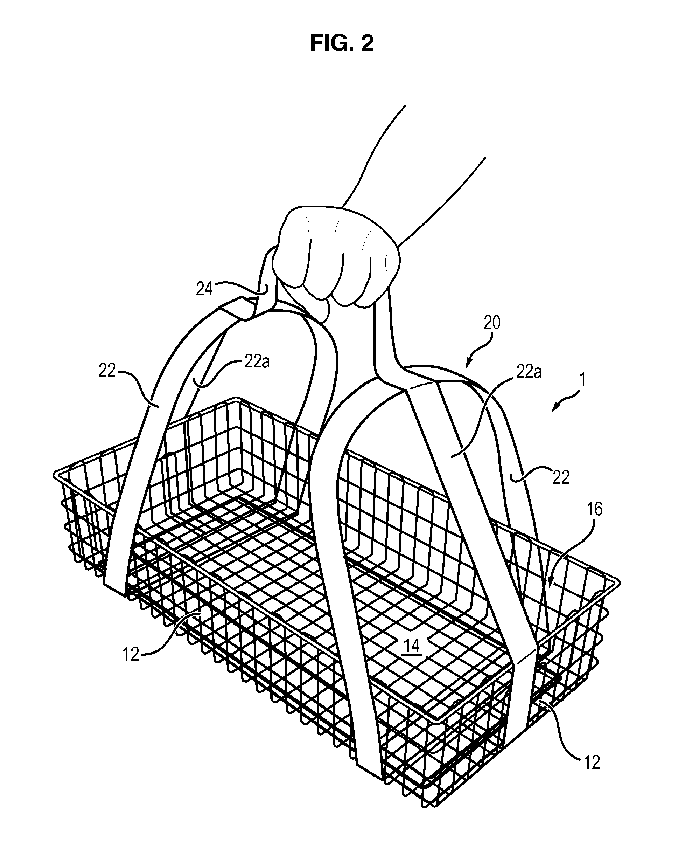

En variante, comme illustré sur la figure 2, les moyens d'accrochage 22 peuvent en outre comprendre deux bandes de maintien latérales 22a, s'étendant transversalement aux boucles fermées 22, dans le prolongement de la poignée de préhension 24, adaptées pour être enfilées sur le panier de stérilisation 1. Les bandes latérales 22a permettent ainsi de maintenir le panier 1 latéralement afin d'éviter qu'il ne glisse et sorte du porte-panier 20. Alternativement, le panier de stérilisation 1 peut comprendre des moyens de retenue du porte-panier 20, adaptés pour l'empêcher de glisser le long du panier de stérilisation 1.

Le porte-panier 20 peut également être formé d'une bande annulaire, disposée contre une face extérieure du fond 14 du panier, une partie de la bande pouvant être agrippée par l'opérateur et ramenée en direction de la partie supérieure 16. La partie de la bande annulaire adjacente au fond 14 joue alors le rôle de moyens d'accrochage 22, tandis que la partie agrippée par l'opérateur joue le rôle de moyen de préhension 24.

En variante, le porte-panier 20 ne comprend qu'une boucle fermée, enfilée autour du panier de stérilisation 1, transversalement à sa partie supérieure 16. La poignée de préhension 24 est alors formée de la partie de la boucle qui s'étend de manière adjacente à la partie supérieure 16, le reste de la boucle formant les moyens d'accrochage 22.

Selon une autre variante de réalisation, le porte-panier 20 est une anse, fixée à deux parois latérales en regard du panier 1. La zone de fixation de l'anse au porte-panier 20 correspond alors à ses moyens d'accrochage 22, tandis que la partie adaptée pour être prise en main par l'opérateur correspond à sa poignée de préhension 24.

Quelle que soit la variante de réalisation, le porte-panier 20 peut être amovible ou destructible en tout ou partie, afin de ne pas gêner l'opérateur au point d'utilisation du panier 1, par exemple au cours de l'intervention chirurgicale. En variante, ils peuvent être fixés solidairement au panier 1.

Par ailleurs, les dimensions et le matériau constitutif du porte-panier 20 sont choisis de sorte que le porte-panier présente une résistance mécanique suffisante pour supporter le poids du panier 1 et des instruments médicaux, à savoir 15 à 20 kg, même après le traitement de stérilisation. Par exemple, le porte-panier 20 peut être réalisé dans un tissu tissé ou non-tissé. Afin de renforcer le porte-panier, l'anse 24 et les boucles fermées 22 peuvent alors être réalisées en doublant les couches de tissu, tandis que les éventuelles bandes de maintien latérales 22a peuvent être réalisées avec une couche simple de tissu. L'anse 24, les boucles fermées 22 et les bandes latérales 22a peuvent ensuite être agrafées ensemble avant d'être stérilisées avec le panier 1.

Nous allons à présent décrire un exemple de procédé d'utilisation d'un panier de stérilisation 1 conforme à l'invention.

Au cours d'une première étape, on désinfecte l'ensemble des instruments médicaux et du panier de stérilisation 1, par exemple dans un laveur ou avec des produits désinfectants.

Les instruments médicaux sont alors disposés dans le panier de stérilisation 1, et le porte-panier 20 est accroché au panier de stérilisation 1.

Typiquement, dans l'exemple de réalisation illustré sur la figure 1, les boucles fermées servant de moyens d'accrochage 22 sont enfilées sur le panier 1 de sorte que la poignée de préhension 24 s'étend transversalement à la partie supérieure du panier 1, en regard du fond 24. Les boucles 22 sont disposées de préférence de manière sensiblement symétrique afin d'équilibrer celui-ci et de le maintenir globalement parallèle au sol lors de son extraction grâce à la poignée de préhension 24. Lorsque le porte-panier 20 comprend des bandes de maintien latérales 22a, les parois latérales 12 du panier 1 sont en outre disposée de préférence de manière à venir en butée contre les bandes latérales 22a.

On enveloppe alors le panier 1 muni du porte-panier 20 dans un emballage adapté permettant la stérilisation du panier 1 et des instruments, par exemple une ou plusieurs feuilles d'emballage, un sachet, des gaines en papier ou plastique, ou de manière générale tout emballage adapté utilisé de manière conventionnelle dans la stérilisation d'instruments médicaux.

Par exemple, on enveloppe le panier 1 dans une feuille d'emballage primaire et une feuille d'emballage secondaire.

Typiquement, ces feuilles d'emballage sont réalisées dans un matériau permettant le passage de l'agent stérilisant choisi (typiquement, de la vapeur d'eau, de l'oxyde d'éthylène ou des rayonnements de radio-stérilisation), la conservation de l'état stérile de l'ensemble formé par le panier 1 muni du porte-panier 20 et comportant les instruments, et la protection des instruments médicaux. Elles doivent en outre résister à des dégradations dues aux conditions de stockage ou de manutention, ainsi qu'au procédé de stérilisation.

Puis, afin de préserver la stérilité des instruments, le panier de stérilisation 1 équipé du porte-panier 20 est enveloppé, selon des pliages conventionnels tels que le pliage « Pasteur » ou « américain », dans les deux feuilles d'emballage.

L'ensemble est alors stérilisé selon les techniques conventionnelles par exemple à la vapeur d'eau, à l'oxyde d'éthylène ou par radio-stérilisation par rayonnement, et le panier de stérilisation 1 ainsi enveloppé peut être entreposé jusqu'à son utilisation.

Au cours d'une intervention chirurgicale, l'opérateur peut alors extraire le panier de stérilisation 1 en limitant les fautes d'asepsie grâce au porte-panier 20.

Pour cela, une personne (qui est de préférence différente de l'opérateur dont les gants doivent rester aseptiques) ouvre les deux feuilles d'emballage enveloppant le panier de stérilisation 1, puis l'opérateur extrait le panier 1 en tirant sur la poignée de préhension 24 afin de soulever le panier de stérilisation 1 sans toucher les feuilles d'emballage. Quel que soit le degré de mémoire de forme (rigidité ou la forme) des feuilles d'emballage, résultant notamment du procédé de stérilisation et de la durée de stockage de l'ensemble, l'extraction du panier 1 à l'aide du porte-panier 20 peut être faite sans risquer de faute d'asepsie, devient aisée et ne requiert plus une vigilance aussi soutenue de l'opérateur.

Il suffit alors de poser le panier 1 au point d'utilisation, par exemple à proximité de l'intervention chirurgicale, et de libérer l'accès à l'espace interne du panier 1 en dégageant la partie supérieure 16 du panier 1. La présence optionnelle des bandes latérales 22a facilite alors la manipulation du panier de stérilisation en l'empêchant de glisser latéralement et de tomber.

Le porte-panier peut être laissé en place durant l'intervention chirurgicale, ou être retiré, par exemple en faisant glisser les boucles 22 vers une extrémité du panier 1. En variante, il est également possible de déchirer ou de couper la poignée de préhension et/ou les moyens d'accrochage.

En fin d'intervention, le porte-panier 20 peut alors être évacué dans un endroit adapté, ou désinfecté pour être réutilisé.

Bien entendu, la présente invention n'est nullement limitée aux formes de réalisation décrites ci-dessus et représentées sur les dessins, mais l'homme du métier saura y apporter de nombreuses variantes et modifications.

## Revendications

1. Panier de stérilisation (1), notamment pour un bloc opératoire, **caractérisé en ce qu'**il est équipé d'un porte-panier (20) comprenant des moyens d'accrochage (22) au panier de stérilisation (1) et une poignée de préhension (24), ledit porte-panier (20) étant non solidaire du panier de stérilisation (1).

2. Panier de stérilisation (1) selon la revendication 1, dans lequel les dimensions et le matériau constitutif du porte-panier (20) sont choisis de sorte qu'il présente une résistance mécanique suffisante pour supporter le poids du panier de stérilisation (1) et d'instruments médicaux.

3. Panier de stérilisation (1) selon l'une des revendications 1 et 2, réalisé dans un tissu tissé ou non-tissé.

4. Panier de stérilisation (1) selon l'une des revendications 1 à 3, dans lequel les moyens d'accrochage (22) comprennent une boucle fermée adaptée pour être enfilée autour du panier de stérilisation (1).

5. Panier de stérilisation (1) selon la revendication 4, dans lequel les moyens d'accrochage (22) comprennent deux boucles fermées.

6. Panier de stérilisation (1) selon la revendication 5, dans lequel le porte-panier (20) comprend en outre des bandes de maintien latérales (22a) s'étendant transversalement aux boucles fermées (22), adaptées pour maintenir le panier de stérilisation (1) en position dans le porte-panier (20).

7. Panier de stérilisation (1) selon l'une des revendications 1 à 6, dans lequel la poignée de préhension (24) comprend une bande fixée sur les moyens d'accrochage (22) de manière à les relier ensemble et à former une anse.

8. Ensemble formé d'un panier de stérilisation (1) équipé d'un porte-panier (20) non solidaire du panier selon l'une des revendications 1 à 7, et enveloppé dans un emballage.

9. Ensemble selon la revendication 8, dans lequel le panier de stérilisation (1) et le porte-panier (20) sont complètement enveloppés dans l'emballage.

10. Ensemble formé d'un panier de stérilisation (1) équipé d'un porte-panier (20) non-solidaire du panier selon l'une des revendications 1 à 7, et comprenant en outre des instruments médicaux stériles logés dans le panier de stérilisation (1).

11. Procédé d'utilisation d'un panier de stérilisation (1) équipé d'un porte-panier (20) non-solidaire du panier selon l'une des revendications 1 à 7, ledit panier étant enveloppé dans un emballage, **caractérisé en ce qu'**il comprend les étapes suivantes :
- ouvrir l'emballage,
- saisir la poignée de préhension (24),
- tirer sur la poignée de préhension (24) de manière à soulever le panier de stérilisation (1) et à l'extraire de l'emballage, et
- poser le panier de stérilisation (1) à un endroit voulu, en dehors de l'emballage.

12. Procédé d'utilisation selon la revendication 10, dans lequel l'emballage comprend une feuille d'emballage primaire et une feuille d'emballage secondaire.

13. Procédé d'utilisation selon l'une des revendications 11 et 12, comprenant en outre une étape au cours de laquelle le porte-panier (20) est jeté après avoir posé le panier de stérilisation (1) à l'endroit voulu.

14. Procédé d'utilisation selon l'une des revendications 11 et 12, comprenant en outre une étape au cours de laquelle le porte-panier (20) est retiré du panier de stérilisation (1) que celui-ci a été posé à l'endroit voulu, puis réutilisé pour porter un nouveau panier (1).
